# DEMANDE DE BREVET EUROPEEN

(11) **EP 4 410 299 A1**
(43) Date de publication de la demande: **07.08.2024**
(21) Numéro de dépôt: 24155627.3
(22) Date de dépôt: 02.02.2024
(51) Int. Cl.: A61K 36/185, A61P 17/00, A23L 33/105, A61K 8/9789, A61P 29/00, A61Q 19/00, A61Q 19/08

(54) **EXTRAIT DE CANNABIS SATIVA CULTIVE IN VITRO**

(30) Priorité: 03.02.2023 FR 2301028
(71) Demandeur: Sativa Toward Health Biotechnologies, 91058 Evry-Coucouronnes Cedex (FR)
(72) Inventeur: KAMINSKY, Naomi, 94240 L'HAY LES ROSES (FR); MAILHAC, Héloïse, 77127 LIEUSAINT (FR)
(74) Mandataire: Regimbeau

(57) **Abrégé**

La présente invention concerne un extrait de tissus racinaires de *C*. *sativa* cultivés *in vitro,* caractérisé en ce que ledit extrait est constitué de biomasse de tissus racinaires, et comprend au moins 0,01% en poids de lignanamide(s) par rapport au poids de l'extrait sec. La présente invention concerne également une composition pharmaceutique, dermatologique, nutraceutique, cosmétique ou vétérinaire comprenant en tant qu'actif l'extrait décrit ci-dessus.

## Description

### DOMAINE DE L'INVENTION

La présente invention concerne des compositions pharmaceutiques, dermatologiques, nutraceutiques, cosmétiques et vétérinaires. Ces compositions sont caractérisées en ce qu'elles contiennent un extrait de tissus racinaires de la plante *Cannabis sativa,* qui ont été cultivés *in vitro* et présentent une composition biochimique spécifique et avantageuse.

### ETAT DE LA TECHNIQUE

La plante de *Cannabis sativa* est cultivée depuis la période du néolithique. Il s'agit d'une plante herbacée annuelle de la famille de Cannabinacées. Plusieurs chémotypes ont été identifiés, basés sur les teneurs en cannabinoïdes majoritaires, pouvant être classés en deux familles de variétés principales : le chanvre ou cannabis fibreux, avec de faible teneur en cannabinoïdes (<5%), et les variétés de cannabis dites médicales ou thérapeutiques, produisant de forte teneur en cannabinoïdes pouvant aller jusqu'à 40%. Parmi ces deux familles, différentes variétés et cultivars peuvent être distingués.

Les utilisations de cette plante sont multiples, elles vont de la confection de textile, de matériau de construction et de papier (chanvre), aux utilisations pharmaceutiques, vétérinaires, nutraceutiques et cosmétiques (chanvre et cannabis médical).

Parmi les nombreux composés d'intérêt extraits de *Cannabis sativa,* les cannabinoïdes sont des composés produits uniquement par cette plante ; ces composés sont très étudiés, en particulier pour leurs propriétés thérapeutiques. Parmi la centaine de cannabinoïdes identifiés, le cannabidiol (CBD) et le delta-9-tetrahydrocannabinol (THC) sont les plus connus.

Mais la plante *Cannabis sativa* comprend également des composés actifs communs avec d'autres plantes, et en particulier les composés d'intérêt suivants (Brenneisen, 2007) :
- Des terpènes, comprenant 5, 10, 15, 20, ou 30 carbones (respectivement dénommés hémiterpènes, monoterpènes, sesquiterpènes, diterpènes, et triterpènes) ;
- Des hydrocarbones, notamment des alcanes ;
- Des sucres, acides gras, alcools, esters ;
- Des flavonoids ;
- Des phytostérols ; et
- Des alcaloïdes, en particulier la cannabisativine et les lignanamides.

Dans la plante de *Cannabis sativa,* toutes les structures anatomiques sont exploitées : les tiges pour leur teneur en fibres de haute qualité, les graines pour leurs propriétés nutritionnelles, notamment leur forte teneur en acides gras et protéines, et naturellement les fleurs et les feuilles qui contiennent l'essentiel des composés cannabinoïdes. Les fruits ont également été étudiés : des cannabisines (A à G) ont été isolées à partir des fruits de *C*. *sativa* (Sakakibara *et al.,* 1995).

Les voies de biosynthèse de ces différents métabolites dans la plante de *Cannabis sativa* ont été étudiées, et sont décrites notamment dans la revue de Flores-Sanchez Σε Verpoorte (2008).

Bien qu'elles ne contiennent que des traces de cannabinoïdes, les racines de la plante *Cannabis sativa* sont utilisées pour des applications thérapeutiques ou nutraceutiques depuis l'antiquité. Malgré cette longue histoire, les racines de *Cannabis sativa* ont été quelque peu délaissées par la médecine moderne, au profit de l'utilisation d'autres parties de la plante. Néanmoins, le potentiel anti-inflammatoire des composés phytochimiques identifiés dans la racine (alcaloïdes, triterpénoïdes, phytostérols) a récemment relancé l'intérêt des professionnels pour les extraits de racine de *Cannabis sativa* (Ryz *et al.,* 2017).

En particulier, l'article de (Kornpointner *et al.,* 2021) présente les résultats d'une analyse de la composition phytochimique d'extraits de racines de *Cannabis sativa,* par chromatographie en phase gazeuse couplée à de la spectrométrie de masse (GC-MS). Cette analyse a permis d'identifier de nouveaux composés jamais décrits dans les racines de *C*. *sativa.* Les triterpénoïdes majoritaires (friedelin et epifriedelinol) ont été quantifiés dans des extraits issus de différents chémovars (« variétés chimiques ») cultivés en pleine terre, extraits selon différentes techniques.

Il apparait que la composition biochimique des extraits de racine est dépendante de nombreux paramètres : chémovar employé, méthode de culture, technologie d'extraction, pour ne citer que quelques-uns.

Des techniques de culture *in vitro* de plantes de *Cannabis sativa* ont été développées.

En particulier, (Whaby *et al.,* 2013) et (Berahmand *et al.,* 2016) ont montré qu'il était possible de transformer des racines de *C. sativa* cultivés *in vitro,* dans des boites de pétri, avec des souches *d'Agrobacterium rhizogenes.* Cette transformation permet la génération de « chevelu racinaire » qui peut être ensuite cultivé dans un milieu nutritif liquide ou solide, afin de générer de la biomasse.

Une autre technique de culture *in vitro* de racines consiste à dédifférencier des cellules végétales issues d'autres organes, et à les traiter par des hormones végétales afin d'obtenir des tissus racinaires dits adventives. (Farag Σε Kaiser, 2015)

Toutefois, ces racines de *C. sativa* cultivées *in vitro* n'ont jamais été caractérisées de manière précise du point de vue de leur composition biochimique.

La présente invention est relative à un nouvel extrait de tissus racinaires de *C. sativa* cultivés *in vitro,* présentant une composition biochimique particulière, et en particulier comprenant au moins une lignanamide.

Plus précisément, la présente invention concerne un extrait de tissus racinaires de *C. sativa* cultivés *in vitro,* caractérisé en ce que ledit extrait est constitué de la biomasse desdits tissus racinaires, et comprend au moins 0,01% en poids de lignanamide(s) par rapport au poids de l'extrait sec.

Or, à ce jour, dans la plante de *Cannabis sativa,* la présence de lignanamides n'avait été observée que dans les graines (Yan *et al.,* 2015) et dans les fruits (Sakakibara *et al.,* 1995).

La présente invention a pour but de fournir un extrait végétal riche en lignanamide(s), et plus particulièrement riche en cannabisine(s), qui soit obtenu à partir d'un tissu végétal disponible et à croissance rapide. La solution proposée est d'utiliser des tissus racinaires de *C. sativa* cultivés *in vitro,* qui présentent une biomasse conséquente, qui croît rapidement, et qui de plus contiennent une teneur élevée en lignanamides.

Un tel extrait de racines de *C. sativa* est caractérisé en ce qu'il est issu d'une culture *in vitro* de tissus racinaires de la plante. Ce procédé de culture permet d'obtenir une biomasse qui est ensuite récoltée, broyée et séchée afin d'obtenir ledit extrait comprenant au moins une lignanamide.

Avantageusement, cet extrait présente des propriétés anti-microbiennes, anti-oxydantes et anti-inflammatoires qui en font un produit actif pouvant être utilisé dans de nombreuses applications, notamment en pharmacie, en dermatologie, en cosmétique et en nutraceutique.

Les inventeurs ont découvert que les extraits de tissus racinaires de *Cannabis sativa* cultivés *in vitro* comprenant au moins une lignanamide présentent des propriétés pharmaceutiques, dermatologiques, cosmétiques, nutraceutiques et vétérinaires, jamais décrites jusqu'à présent.

### EXPOSE DE L'INVENTION

La présente invention se rapporte à un extrait de tissus racinaires de *C. sativa* cultivés *in vitro* comprenant au moins une lignanamide. Ledit extrait est caractérisé en ce qu'il est constitué de la biomasse desdits tissus racinaires, et qu'il comprend au moins 0,01% en poids de lignanamides par rapport au poids de l'extrait sec.

Par souci de concision, dans la présente demande, les expressions « extrait de tissus racinaires de *C. sativa* cultivés *in vitro » ou* « extrait de tissus racinaires de *C*. *sativa* » ou encore « extrait de tissus racinaires selon l'invention » sont utilisées indifféremment et désignent toutes un « extrait de tissus racinaires de *C. sativa* cultivés *in vitro* constitué de biomasse comprenant au moins 0,01% en poids de lignanamide(s) par rapport au poids de l'extrait sec ».

L'invention concerne également des procédés d'obtention de cet extrait de tissus racinaires de *C. sativa* cultivés *in vitro* comprenant au moins 0,01% en poids de lignanamide(s) par rapport au poids de l'extrait sec.

Le premier procédé de préparation de l'extrait est caractérisé en ce qu'il comprend la transformation de plantules avec une bactérie *Agrobacterium rhizogenes* pour l'obtention d'un chevelu racinaire, cultivé dans un milieu nutritif, puis séché et broyé.

Le second procédé de préparation d'un extrait de tissus racinaires de *C. sativa* est caractérisé en ce qu'il comprend la dédifférentiation d'un explant de *C. sativa* en cal, puis la différentiation du cal en tissus racinaires adventives, cultivés dans un milieu nutritif, puis séchés et broyés.

Enfin, l'invention se rapporte également à des compositions pharmaceutique, dermatologique, nutraceutique, cosmétique ou vétérinaire, comprenant en tant qu'actif cet extrait de tissus racinaires de *C. sativa* cultivés *in vitro* comprenant au moins 0,01% en poids de lignanamide(s) par rapport au poids de l'extrait sec.

### DESCRIPTION DES FIGURES

La figure 1 représente une culture sur milieu solide de tapis racinaire chevelus de cannabis sativa agro-transformé âgés de 5 semaines.
La figure 2 représente une culture en milieu liquide de tissus racinaires chevelus de cannabis sativa agro-transformés en milieu liquide à différents stades de croissance : (A) Biomasse le jour de sa mise en suspension, (B) biomasse après cinq jours de croissance, (C) biomasse après 8 jours de croissance.
La figure 3 montre la culture en milieu liquide de tissus racinaires de cannabis sativa agro-transformés en bioréacteur. (A) Biomasse dans le bioréacteur, (B) Biomasse récoltée hors du bioréacteur.
La figure 4 représente une culture sur milieu solide de tissus de racines adventives différenciées de *Cannabis sativa,* à différent stade de différenciation.
La figure 5 représente une culture en milieu liquide de tissus de racines adventives différenciées de *Cannabis sativa* en milieu liquide, à différent stade de croissance.
La figure 6 présente des photos de la biomasse de tissus racinaires de *Cannabis sativa* agro-transformé, séchée et broyée au mortier. (A) Biomasse séchée, (B) Biomasse broyée.
La figure 7 montre les profils spectraux RMN (Résonance Magnétique Nucléaire) des deux fractions apolaire (UP) et polaire (LOW) obtenues après une extraction par solvant des composés biochimiques contenus dans l'extrait de tissus racinaires de l'invention.
La figure 8 présente les profils LC/MC des deux fractions apolaire et polaire obtenues après extraction par solvant des composés biochimiques contenus dans l'extrait de tissus racinaires de l'invention.
La figure 9 présente une Analyse de Composant Principale (ACP) montrant les différences de potentiels antimicrobien et antioxydant de l'extrait de tissus racinaires de l'invention (STH1) et d'un tissu racinaire naturel (STH2), avec le DMSO utilisé en témoin négatif, et un témoin positif E.
La figure 10 présente une « clusterisation ascendante » regroupant les différents extraits analysés (STH1, STH2, DMSO et témoin E) selon leur potentiel antimicrobien et antioxydant en groupe à faible potentiel (cluster 1), potentiel modéré (cluster 2) et potentiel fort (cluster 3).
La figure 11 présente une confirmation de l'activité antimicrobienne de l'extrait de tissus racinaire de l'invention (STH1) comparé à l'extrait de tissu racinaire naturel (STH2), par mesure de l'inhibition de la croissance de *Saccharomyces cerevisiae* en fonction du temps. Le DMSO est utilisé en témoin négatif et le témoin E en témoin positif.
La figure 12 présente une confirmation de l'activité antioxydante du tissu racinaire de l'invention (STH1) comparé à un tissu racinaire naturel (STH2), par l'évaluation du potentiel anti-radicalaire en équivalent acide gallique (µM) de 10 µg de chacun des extraits.

### DESCRIPTION DETAILLEE DE L'INVENTION

La présente demande concerne un extrait de tissus racinaires de *C. sativa* cultivés *in vitro* comprenant au moins un alcaloïde, et plus particulièrement au moins une lignane, et notamment au moins une lignanamide, et en particulier 0,01% en poids de lignanamide(s) par rapport au poids de l'extrait sec.

Au sens de l'invention, on désigne par *Cannabis sativa* ou *C. sativa* la plante ainsi nommée, également désignée par le terme « chanvre ». Ce terme inclut toutes les variétés et cultivars connus.

Le terme « tissus racinaires » désigne des tissus végétaux différenciés en racines, à savoir la partie souterraine de la plante lorsque celle-ci est cultivée en champ. Ces tissus végétaux présentent les mêmes caractéristiques et fonctions que les racines, en particulier assurer l'approvisionnement en eau et en éléments minéraux de la plante, permettre l'ancrage de la plante sur le substrat et stocker les réserves en assimilats.

Au sens de l'invention, l'expression « cultivés *in vitro* » désigne le fait que les tissus racinaires sont cultivés hors sol, en l'absence de terre, et sont en particulier cultivés dans des milieux nutritifs adaptés, solides ou liquides en environnement contrôlé. Cette culture *in vitro* est réalisée dans des milieux bien connus de la personne de l'art, tel que par exemple le milieu MS (pour milieu de Murashige and Skoog).

Le terme « extrait » désigne le fait que les tissus racinaires de *C. sativa* obtenus par culture *in vitro,* également désignés par le terme « biomasse », sont soumis à diverses étapes de procédé (notamment séchage et broyage) afin d'obtenir ledit extrait de tissus racinaires, qui est un extrait sec, dépourvu d'eau.

Plus précisément, l'extrait selon l'invention est constitué de la biomasse desdits tissus racinaires de *C. sativa* récoltée.

Au sens de l'invention, l'expression « au moins une » signifie « une ou plusieurs ».

Au sens de l'invention, l'expression « au moins 0,01% en poids de lignanamide(s) par rapport au poids de l'extrait sec » signifie une proportion relative de l'ensemble des lignanamides de l'extrait, en poids par rapport au poids total de l'extrait sec. Il est entendu que cet « ensemble des lignanamides » comprend une ou plusieurs lignanamides, et en particulier une ou plusieurs cannabisines.

Un alcaloïde est une substance organique d'origine végétale, contenant au moins un atome d'azote dans la molécule, en grande majorité hétérocyclique. Les alcaloïdes ont généralement une puissante action toxique ou thérapeutique (les composés tels que caféine, morphine, quinine, sont des alcaloïdes).

Les lignanes sont des substances organiques d'origine végétale, dont la formule générale (1) est représentée ci-dessous :

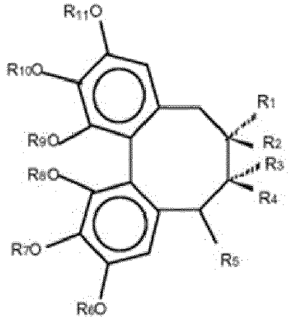

Les lignanes comprennent au moins deux groupements « phénol » caractérisés par la présence d'un cycle aromatique hydrocarboné et d'un ou plusieurs groupes hydroxyles (OH), ici désignés par le nom de monolignol. Il existe trois principaux monolignols : l'alcool coniférylique, l'alcool sinapylique et l'alcool *para*-coumarylique.

De très nombreux lignanes ont été répertoriées, qui diffèrent par le type de liaison entre les deux unités et les modifications qui interviennent après la dimérisation. Les propriétés anti-oxydantes et anti-inflammatoires des lignanes ont été rapportées, par exemple dans la demande de brevet WO 2021/021744.

Les lignanamides représentent une sous-catégorie des lignanes. Elles dérivent des acides aminés tyrosine et phénylalanine, et sont issues d'un couplage oxydatif avec des amides d'acide hydroxy-cinnamique comme intermédiaires. La revue (Leonard *et al.,* 2020) présente leur voie de biosynthèse à partir des précurseurs N-caffeoylthyramine et N-feruoylthyramine.

Les lignanamides incluent les cannabisines A, B, C, D, E, F, G, H, I, J, K, L, M, N et O, ainsi que la grossamide. (Sakakibara *et al.,* 1995) décrit l'identification des cannabisines A à G dans les fruits de la plante *Cannabis sativa.*

La formule (2) ci-dessous est celle de la cannabisine F, de numéro CAS 163136-19-4 :

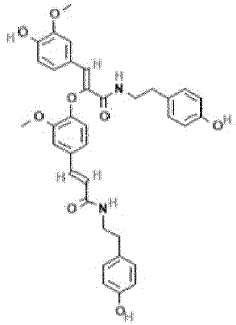

La formule (3) ci-dessous est celle de la cannabisine G, de numéro CAS 163136-20-7:

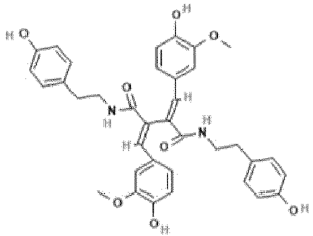

Les lignanamides présentent des propriétés anti-inflammatoires et anti-oxydantes. Ces propriétés ont notamment été mises en évidence pour la cannabisine F et la grossamide issues des graines de *Cannabis sativa* ; pour la mélongénamide C issue des racines de *Solanum melongena* ; et pour la cannabisine B issue des coques des graines de *Cannabis sativa* (Leonard *et al.,* 2020, voir notamment le tableau 2). L'article (Wang et al., 2019) décrit les propriétés anti-inflammatoires de la cannabisine F.

La présente invention concerne un extrait de tissus racinaires de *C. sativa* cultivés *in vitro* comprenant au moins une lignanamide, en particulier choisie parmi les cannabisines A, B, C, D, E, F, G, H, I, J, K, L, M, N et O, la grossamide et leurs mélanges, dans une proportion de 0,01% en poids de lignanamides par rapport au poids de l'extrait sec.

Plus spécifiquement, l'extrait de tissus racinaires de *C. sativa* est caractérisé en ce que ladite au moins une lignanamide est au moins une cannabisine, en particulier choisie parmi les cannabisines A, B, C, D, E, F et G et leurs mélanges, dans une proportion de 0,01% en poids de cannabisine(s) par rapport au poids de l'extrait sec.

De manière préférée, ledit extrait de tissus racinaires comprend au moins une lignanamide choisie parmi les cannabisines D, F et G (respectivement de numéro CAS 144506-19-4, 163136-19-4 et 163136-20-7) et leurs mélanges, dans une proportion de 0,01% en poids de cannabisines D, F ou G par rapport au poids de l'extrait sec.

L'extrait selon l'invention pourra comprendre une, deux, trois, quatre, cinq, six ou plus de lignanamides/cannabisines différentes.

En particulier, l'invention a pour objet un extrait de tissus racinaires de *Cannabis sativa* cultivés *in vitro* riche en lignanamides, typiquement contenant au moins 0,01% en poids de lignanamides, et notamment de cannabisines, par rapport au poids de l'extrait sec.

Avantageusement, l'extrait de tissus racinaires de *C. sativa* cultivés *in vitro* contient au moins 0,1%, avantageusement au moins 1%, en poids de lignanamides par rapport au poids total de l'extrait sec.

Avantageusement, l'extrait de tissus racinaires de *Cannabis sativa* cultivés *in vitro* contient également d'autres composés d'intérêt et notamment des phytostérols, des dérivés de tyramine tels que N-coumaroyltyramine, N-feruloyltyramine, N-caffeoyltyramine, des triterpènes ainsi que d'autres alcaloïdes tels que la cannabisativine ou l'anhydrocannabisativine.

De manière avantageuse, l'extrait de tissus racinaires de l'invention contient, outre au moins 0,01% en poids de lignanamides par rapport au poids de l'extrait sec, au moins un autre composé actif choisi parmi la N-coumaroyltyramine, la N-feruloyltyramine, la N-caffeoyltyramine, la cannabisativine, ou tout mélange de ces composés.

Au sens de l'invention, le terme « biomasse » désigne une masse de matière vivante, ici de matière végétale, représentant un ensemble de matières organiques susceptibles de devenir une source d'énergie ou de matière.

L'extrait de tissus racinaires cultivés *in vitro* selon l'invention est constitué de biomasse de tissus racinaires, avantageusement séchée et broyée après récolte.

Lorsque lesdits tissus racinaires ont subi une transformation par une bactérie de type *Agrobacterium Rhizogenes,* les tissus racinaires adoptent une structure particulière qui est désignée « chevelu racinaire ». Il s'agit d'une prolifération racinaire importante liée à un déséquilibre hormonal des racines, qui est considérée comme une maladie par les producteurs. Toutefois dans le cadre d'une culture *in vitro,* ayant pour but de produire de la biomasse en quantité sur une durée minimale, cette prolifération racinaire est appréciée.

Selon cette mise en oeuvre, ledit extrait de tissus racinaires de *C. sativa* est constitué de biomasse de chevelu racinaire de *C. sativa* cultivé *in vitro,* séchée et broyée.

Selon un autre aspect, la présente invention concerne un extrait de chevelu racinaire de *C. sativa,* obtenu par transformation des tissus racinaires de *C. sativa* par une bactérie *Agrobacterium* suivie d'une culture *in vitro.*

Préférentiellement, ladite bactérie *Agrobacterium* est *Agrobacterium rhizogenes,* et elle ne contient pas d'ADN exogène.

### Procédés de préparation de l'extrait de tissus racinaires selon l'invention

La présente invention concerne également des procédés de préparation d'un extrait de tissus racinaires de *C. sativa* cultivés *in vitro.*

Le fait de cultiver des tissus racinaires de *C*. *sativa in vitro* permet d'obtenir des tissus racinaires présentant des propriétés tout à fait intéressantes, notamment des tissus comprenant au moins une lignanamide, et plus particulièrement des tissus riches en lignanamides, c'est-à-dire que l'extrait sec comprend au moins 0,01% en poids de lignanamides par rapport à son poids total.

Un autre avantage de ces procédés est la disponibilité du tissu végétal cultivé *in vitro,* qui permet de s'affranchir de l'étape de récolte de la plante lorsque celle-ci est cultivée en champ.

De plus, la culture en condition contrôlé et stérile permet d'éviter les contaminations par les pesticides ou les métaux lourds qui peuvent être présents dans les sols.

Par ailleurs, contrairement à la culture des plantes en milieu semi-contrôlé telles que les cultures en intérieur ou en serre, la consommation d'énergie et d'eau est nettement réduite. En effet, les racines isolées sont cultivées sans lumière - contrairement aux plantes entières qui nécessite au moins 200 watts par mètre carré sur une photopériodicité de 18h en lumière en moyenne. La culture de racine isolé nécessite l'ajout de milieu liquide régulièrement (environ 10% du volume par semaine), mais cela représente peu d'eau par rapport à une culture de plante entière, qui nécessite 1,5 Litre d'eau par plante par jour en moyenne sur toute la durée du cycle de croissance de la plante.

Un avantage additionnel de ces procédés est la vitesse de croissance des tissus végétaux *in vitro,* qui permet de produire de la biomasse en quantité, en un minimum de temps (3 à 5 semaines de culture, selon l'unité et le volume de production).

Enfin, ce type de culture est continu et non dépendant des saisons ou du cycle de croissance d'une plante entière (en moyenne 2 à 4 cycle de croissance par an).

Selon une première mise en oeuvre, le procédé de préparation d'un extrait de tissus racinaires de *C. sativa* comprend la transformation de plantules de *C. sativa* avec une bactérie *Agrobacterium rhizogenes* pour l'obtention d'un chevelu racinaire, cultivé dans un milieu nutritif, puis séché et broyé.

Selon une mise en oeuvre particulière, ce premier procédé comprend les étapes successives suivantes :
a) Mise en culture de graines de *C. sativa* dans un milieu de germination permettant le développement de plantules ;
b) Transformation des tissus racinaires desdites plantules avec une bactérie *Agrobacterium rhizogenes* pour l'obtention d'un chevelu racinaire ;
c) Culture *in vitro,* dans un milieu nutritif liquide, sous agitation, dudit chevelu racinaire de *C*. *sativa* ;
d) Prélèvement, séchage et broyage dudit chevelu racinaire ainsi généré.

Les différents milieux utilisés dans ce procédé sont bien connus de l'Homme du métier. On peut citer notamment comme exemple de milieu nutritif le milieu MS enrichi en saccharose.

L'étape (b) de transformation des plantules avec une bactérie *Agrobacterium rhizogenes* est bien connue de l'Homme du métier. En particulier, (Wahby et al., 2013) ont présenté un protocole d'infection de la plante *Cannabis sativa* par *Agrobacterium rhizogenes,* réalisé *in vitro* sur cinq différentes variétés de cette espèce.

Dans le procédé selon la présente invention, la bactérie *Agrobacterium rhizogenes* ne contient pas d'ADN exogène. En effet, la transformation des tissus racinaires a pour but d'obtenir la croissance de tissus dits « chevelu racinaire », mais non de transformer lesdits tissus génétiquement de manière à faire exprimer des protéines codées par des séquences nucléotidiques exogènes.

Ainsi, dans le procédé selon l'invention, seul l'ADN-T de *Agrobactreium rhizogenes,est* introduit dans les cellules des tissus racinaires transformés. En particulier l'ADN-T est composé des éléments *rol A, B, C et D* qui sont des facteurs de croissance et de différenciation des cellules, les gènes *iaaM* et *iaaH* qui sont responsables de la synthèse d'auxine.

L'étape (c) de culture *in vitro* dure au moins 1, 2, 3, 4, 5 ou 6 jours, ou 7 jours, ou 14 jours, ou 21 jours, ou 28 jours, en fonction de la quantité *l* du volume de chevelu racinaire souhaitée.

Les conditions de cette culture (agitation, température, lumière) seront aisément déterminées par la personne de l'art sur la base de ses connaissances générales.

L'étape (d) de prélèvement, séchage et broyage des tissus racinaires sera réalisée selon les protocoles bien connus de la personne de l'art.

En ce qui concerne le séchage, il sera de préférence réalisé par dessication (en utilisant un dessiccateur), par lyophilisation, par évaporation ou par chauffage.

En particulier, le broyage pourra être réalisé grâce à un mortier ou tout autre équipement de broyage tel qu'un broyeur à bille, un broyeur ultracentrifuge, ou avec un dispositif de cryobroyage.

L'extrait séché et broyé est ensuite conservé dans un pot, de préférence à une température de 4°C.

Selon une seconde mise en oeuvre, le procédé de préparation d'un extrait de tissus racinaires de *C. sativa* comprend la dédifférentiation d'un explant de *C. sativa* en cal, puis la différentiation du cal en tissus racinaires adventis, cultivés dans un milieu nutritif, puis séchés et broyés.

Un tel procédé est bien connu de la personne de l'art et est notamment décrit dans l'article de (Farag Σε Kayser, 2015). L'explant pourra être toute partie de la plante, et sera de préférence stérilisé préalablement à sa mise en culture.

Les expressions « tissus racinaires adventis » ou « racines adventives » désignent des racines qui apparaissent le long d'une tige, spécialement dans les entre-noeuds.

Selon une mise en oeuvre particulière, ce second procédé comprend les étapes suivantes :
a) Mise en culture *in vitro* d'un explant de *C. sativa* sur un milieu nutritif solide ;
b) Induction de callogenèse par ajout de phytohormones audit milieu nutritif solide ;
c) Culture du cal obtenu à l'étape (b) dans un milieu nutritif liquide comprenant un mélange de phytohormones spécifique, sous agitation, pour induire la formation de tissus racinaires adventis ;
d) Prélèvement, séchage et broyage desdits tissus racinaires ainsi générés.

L'étape (b) d'induction de callogenèse est avantageusement réalisée par l'ajout des cytokines et/ou d'auxines.

L'étape (c) de culture *in vitro* dure au moins 1, 2, 3, 4, 5, 6 ou 7 jours, ou au moins 14 jours, ou au moins 21 jours, ou au moins 28 jours, ou au moins 35 jours, ou encore au moins 42 jours, en fonction de la quantité / du volume de tissus racinaires adventis souhaitée.

Les conditions de cette culture (agitation, température, lumière) seront aisément déterminées par la personne de l'art sur la base de ses connaissances générales.

L'étape (d) sera réalisée comme précédemment décrit dans le cadre du premier procédé.

Ces deux procédés sont décrits sur la base des étapes essentielles à leur réalisation. IL est néanmoins entendu que des étapes optionnelles, bien connues de la personne de l'art, pourront être réalisées en surplus.

En particulier, une étape d'extraction de certaines molécules (notamment des lignanamides) peut être insérée avant ou après le séchage et le broyage de l'extrait. Une telle extraction peut être réalisée à l'aide d'un solvant adapté ou par extraction mécanique.

Parmi les solvants adaptés, on peut citer l'eau, l'éthanol, l'acétonitrile, la glycérine, les huiles végétales, l'hexane, le butanol, l'acétate d'éthyle, le dichlorométhane. L'extraction peut être réalisée par des solvants sous-pression, par fluides supercritiques (les plus courant sont l'eau et le CO2 supercritique), par distillation, diffusion, infusion, macération.

Parmi les techniques d'extraction mécanique on peut citer la sonication, les micro-ondes, l'extrusion, les champs électriques pulsés, et la détente instantanée contrôlée.

Avantageusement, ces procédés de préparation de l'extrait de tissus racinaires de C. *sativa* cultivés *in vitro* permettent de concentrer les lignanamides présentes dans ces tissus racinaires, de manière à ce que cet extrait contienne au moins 0,01% en poids de lignanamides, de préférence au moins 0,1% en poids et plus avantageusement au moins 1% en poids par rapport au poids total de l'extrait sec.

### Compositions comprenant l'extrait de tissus racinaires de C. sativa selon l'invention

L'invention a également pour objet une composition comprenant en tant qu'actif un extrait de tissus racinaires de *C. sativa* tel que défini ci-dessus ou tel qu'obtenu par l'un des procédés de l'invention, éventuellement en association avec un excipient approprié et/ou un véhicule.

La présente invention concerne une composition pharmaceutique, une composition dermatologique, une composition nutraceutique et une composition cosmétique.

Ladite composition est de préférence formulée pour être administrée par voie topique externe ou orale.

La composition selon l'invention comprend en tant qu'actif une quantité efficace d'extrait de tissus racinaires de *C. sativa* selon l'invention. Au sens de l'invention, on entend par « quantité efficace », la quantité nécessaire et suffisante pour obtenir l'effet recherché selon l'invention, à savoir l'activité pharmacologique, dermatologique, nutraceutique ou cosmétique dudit extrait après son administration à un individu. Cette « quantité efficace » dépend de la nature de l'extrait de tissus racinaires, de l'effet recherché, de l'individu à qui est administré la composition de l'invention et de sa condition physiopathologique. Une quantité efficace peut aisément être déterminée par toute méthode connue de l'homme de métier, notamment, par exemple, au travers d'essais cliniques.

Naturellement, une telle composition pourra comprendre au moins un autre composé actif, ainsi qu'un ou des excipients et un ou des véhicules pharmaceutiquement acceptables.

A titre d'excipient, on peut citer le carbonate de magnésium, le dioxyde de titane, le lactose, le mannitol et d'autres sucres, le talc, les lactoprotéines, la gélatine, l'amidon, la cellulose et ses dérivés, les huiles animales et végétales, le polyéthylène glycol, la glycérine et des solvants tels que l'eau et des mono-ou polyalcools tels que le glycérol.

De nombreux véhicules pharmaceutiquement acceptables peuvent être utilisés, par exemple, l'eau, l'eau tamponnée, une solution saline, une solution de glycine, ainsi que des agents nécessaires pour reproduire les conditions physiologiques comme par exemple des agents tampons et ajusteurs de pH, des surfactants comme l'acétate de sodium, le lactate de sodium, le chlorure de sodium, le chlorure de potassium, ou le chlorure de calcium, cette liste n'étant pas limitative.

Une composition selon l'invention peut comprendre également des additifs, des liants, des agents de gonflement ou lubrifiants, ou des agents de solubilisation habituellement mis en oeuvre dans le domaine.

La composition selon la présente invention peut en outre comprendre au moins un adjuvant pharmaceutique ou cosmétique connu de l'homme du métier, choisi parmi les épaississants, les conservateurs, les parfums, les colorants, des filtres chimiques ou minéraux, les agents hydratants, les eaux thermales, etc.

L'invention concerne en particulier une composition pharmaceutique et/ou dermatologique, comprenant en tant qu'actif un extrait de tissus racinaires de *C. sativa* tel que défini ci-dessus ou tel qu'obtenu par l'un des procédés de l'invention.

Cette composition pharmaceutique selon l'invention peut être formulée sous toute forme galénique appropriée telle qu'une solution injectable, des comprimés, des gélules, ou des patchs transdermiques.

En ce qui concerne la composition dermatologique selon l'invention, elle sera formulée selon une forme administrable par voie topique, et donc sous forme de crème, onguent ou gel.

Cette composition pharmaceutique et/ou dermatologique pourra être stérilisée par des techniques de stérilisation bien connues de la personne de l'art.

L'invention concerne également une composition nutraceutique, définie comme étant une composition prise par voie orale et ayant un effet pharmaceutique et/ou cosmétique, comprenant en tant qu'actif un extrait de tissus racinaires de *C. sativa* cultivés *in vitro.*

L'invention concerne également un complément alimentaire comprenant en tant qu'actif un extrait de tissus racinaires de *C. sativa* cultivés *in vitro.*

L'invention concerne également une composition cosmétique comprenant, en tant qu'actif, un extrait de tissus racinaires de *C. sativa* tel que défini ci-dessus ou tel qu'obtenu par le procédé de l'invention.

Par « composition cosmétique », on entend au sens de la présente invention un produit tel que défini dans la Directive 93/35/CEE du 14 juin 1993 modifiant la Directive 76/768/CEE.

Selon un autre aspect de l'invention, la composition comprend en outre au moins un autre composé actif en plus de l'extrait de tissus racinaires de *C. sativa* cultivés *in vitro.*

Cet autre composé peut être en particulier choisi parmi des actifs classiquement utilisés en dermatologie, en pharmaceutique, en nutraceutique ou en cosmétique et connus de l'Homme de l'art, tels que les émollients, les actifs hydratants, les kératorégulateurs, les kératolytiques, les agents cicatrisant et/ou restructurant de la barrière cutanée, les agonistes PPAR, RXR ou LXR, les agents sébo-régulateurs, les agents anti-irritants et/ou anti-inflammatoires et/ou apaisants, les agents anti-oxydants, les agents anti-âge, les agents dépigmentants ou hypopigmentants, les agents pigmentants, les agents lipolytiques ou inhibiteurs de la lipogénèse ou encore les agents anti-cellulite ou amincissants, les filtres et écrans solaires minéraux ou organiques (pigmentaires ou ultrafins), les composés antifongiques, les conservateurs, les agents anti-bactériens, les pré et probiotiques, les antibiotiques, et les composés immnumodulateurs.

### Voies d'administration

(Ryz et al., 2017) enseigne que la plupart des compostions à base d'extraits de racines de *Cannabis sativa* sont des compositions adaptées pour une administration par voie topique.

La composition selon l'invention peut être formulée sous la forme de différentes préparations adaptées à une administration topique, à une administration orale, rectale, vaginale, nasale, auriculaire ou bronchique, ainsi qu'à une administration parentérale.

La composition selon l'invention est avantageusement formulée sous la forme de différentes préparations adaptées à une administration topique, plus particulièrement pour application sur la peau, et/ou les phanères et/ou les muqueuses, de l'Homme ou de l'animal.

Dans le cadre d'une utilisation pharmaceutique, ou dermatologique, ou cosmétique, la composition sera avantageusement formulée sous la forme d'une préparation adaptée à une administration topique.

Selon cette variante, les différentes préparations sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Dans le cadre d'une utilisation à visée nutraceutique ou cosmétique (de type «cosmet-food »), la composition sera avantageusement formulée sous la forme d'une préparation adaptée à une administration orale.

Selon cette deuxième variante, les différentes préparations sont adaptées à une administration orale ; l'extrait de tissus racinaires de *Cannabis sativa* pouvant entrer soit dans un complément alimentaire soit dans une composition nutraceutique. La composition nutraceutique peut se présenter sous forme de l'extrait de tissus racinaires de *Cannabis sativa* en tant que tel ou bien sous forme de gélules ou de capsules molles de gélatine ou végétales dans le cadre de la présente invention.

### Utilisations de l'extrait de tissus racinaires selon l'invention

L'invention a également pour objet l'utilisation d'un extrait de tissus racinaires de C. *sativa* cultivés *in vitro* tel que décrit ci-dessus pour la fabrication d'une composition pharmaceutique, dermatologique, nutraceutique, cosmétique ou vétérinaire.

Comme préalablement indiqué, l'extrait selon l'invention présente des propriétés anti-microbiennes, anti-inflammatoires et anti-oxydantes tout à fait remarquables, qui peuvent être mises à profit dans des utilisations pharmaceutique, dermatologique, nutraceutique cosmétique ou vétérinaire.

Selon un premier aspect, l'invention concerne un extrait de tissus racinaires de *C. sativa* tel que décrit ci-dessus ou tel qu'obtenu par l'un des procédés mentionnés ci-dessus, ou une composition pharmaceutique et/ou dermatologique le comprenant, pour son utilisation dans la prévention et/ou le traitement des réactions, troubles ou pathologies de la peau et/ou des muqueuses et/ou des phanères, en particulier dans la prévention et/ou le traitement des altérations du tissu dermique, de l'Homme ou de l'animal.

On peut citer comme exemple d'altération du tissu dermique les déséquilibres de la matrice dermique tels les rides, les problèmes de cicatrisation, la fermeté ou l'élasticité de la peau.

Ainsi, l'extrait selon l'invention ou la composition pharmaceutique et/ou dermatologique le comprenant est également avantageusement utilisée dans la prévention du vieillissement, en particulier du vieillissement intrinsèque ou encore du vieillissement photo-induit et de la peau vergeturée.

En particulier, l'extrait selon l'invention ou la composition pharmaceutique et/ou dermatologique le comprenant est destiné à la prévention et/ou au traitement des réactions ou pathologies de type allergiques, inflammatoires, irritatives ou des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

En particulier, l'extrait selon l'invention ou la composition pharmaceutique et/ou dermatologique le comprenant est destiné à une utilisation dans la prévention et le traitement des réactions inflammatoires ou irritatives de la peau et/ou des muqueuses et/ou des phanères.

Avantageusement, l'extrait selon l'invention ou la composition pharmaceutique et/ou dermatologique le comprenant est utilisé(e) pour la prévention et/ou le traitement des réactions, troubles ou pathologies de la peau et/ou des muqueuses et/ou des phanères, et en particulier :
- de la peau, telles que l'acné, la rosacée ou érythrocouperose, le psoriasis, la dermite du siège, la dermatite atopique, l'eczéma, la dermatite de contact, la dermatite irritative, la dermatite allergique, la dermite séborrhéique (croûte de lait), le psoriasis, le zona, la peau sensible, la peau réactive, la peau sèche (xérose), la peau déshydratée, la peau avec rougeur, l'érythème cutanée, la peau photosensibilisée, la peau pigmentée (mélasma, pigmentation post-inflammatoire...), la peau dépigmentée (vitiligo), les dartres, les gerçures, les piqûres, les crevasses en particulier des seins, les coups de soleil, les inflammations dus aux rayons de toutes sortes, les irritations par agents chimiques, physiques (par exemple contrainte de tension pour les femmes enceintes), bactériologiques, fongiques ou viraux, parasitaires (poux, gale, teigne, acariens, dermatophytes), radiologiques ou par déficit de l'immunité innée (peptides antimicrobiens) ou acquise (cellulaire, humorales, cytokines), et/ou
- des muqueuses telles que les gencives et parodontes pouvant présenter des gingivites (gencives sensibles des nouveaux nés, problèmes d'hygiène, dus au tabagisme ou autres), des parodontopathies, ou des muqueuses génitales pouvant présenter des irritations des sphères génitales maies ou femelles externes ou internes et/ou
- des phanères tels que les ongles (ongles cassants, fragiles ...) et des cheveux (alopécie, pellicules, hirsutismes, dermatites séborrhéiques, folliculites) immatures, normaux ou matures, présentant en particulier des troubles du cuir chevelu tels que les alopécies (ou pelade) androgénétiques, aiguës, localisées, cicatricielles, congénitales, occipitales du nourrisson, aerata, dues à la chimiothérapie/radiothérapie ou encore l'effluvium télogène, l'effluvium anagène, la dystrophie pilaire, la trichotillomanie, la teigne ou les pellicules grasses ou sèches, la kératose actinique, le lichen plan polaire, ou le lupus érythémateux discoïde.

Ce procédé pourra être mis en oeuvre chez un être humain (application thérapeutique) ou chez un animal (application vétérinaire).

Selon un deuxième aspect, l'invention concerne l'utilisation nutraceutique ou cosmétique d'un extrait de tissus racinaires de *C. sativa* tel que décrit ci-dessus ou tel qu'obtenu par l'un des procédés mentionnés ci-dessus, ou d'une composition nutraceutique ou d'une composition cosmétique ou d'une composition vétérinaire telles que décrites ci-dessus.

Cette utilisation nutraceutique ou cosmétique aura pour but d'améliorer la fermeté, l'élasticité ou la tonicité de la peau, ou de prévenir le manque de fermeté ou de tonicité ou d'élasticité de la peau.

Cette utilisation nutraceutique ou cosmétique aura également pour but un effet antivieillissement ou anti-âge de la peau, des phanères ou des muqueuses.

Une autre utilisation nutraceutique ou cosmétique de l'extrait selon l'invention sera pour le soin ou l'hygiène de la peau et/ou des muqueuses et/ou des phanères tels que les cheveux.

Une autre utilisation nutraceutique de l'extrait selon l'invention sera pour promouvoir le confort de l'intestin, l'équilibre du microbiote et le renforcement de l'immunité.

Cette utilisation concerne à la fois les êtres humains et les animaux (application vétérinaire).

Selon un troisième aspect, l'invention concerne un procédé de soin cosmétique de la peau et/ou des phanères et/ou des muqueuses, en vue d'améliorer leur état et/ou leur aspect, comprenant l'administration ou l'application d'une composition nutraceutique ou cosmétique ou vétérinaire ou d'un extrait selon la présente invention.

Ce procédé pourra être mis en oeuvre chez un être humain (Homme) ou chez un animal (application vétérinaire).

Dans un mode de réalisation du procédé cosmétique selon l'invention, la composition nutraceutique ou cosmétique ou l'extrait selon l'invention est utilisé en vue d'améliorer la fermeté, l'élasticité ou la tonicité de la peau, ou de prévenir le manque de fermeté ou de tonicité ou d'élasticité de la peau, on encore pour synthétiser et/ou protéger la matrice extracellulaire, ou encore pour prévenir et/ou traiter les altérations du tissu dermique.

Dans un autre mode de réalisation du procédé cosmétique selon l'invention, la composition nutraceutique ou cosmétique ou l'extrait selon l'invention est utilisé comme agent antivieillissement ou anti-âge de la peau, des phanères ou des muqueuses, en particulier contre le vieillissement intrinsèque ou extrinsèque, notamment comme agent photovieillissement ou agent anti-UV, ou encore comme agent pour lutter contre l'accumulation du tissu adipeux et la peau avec cellulite.

De manière particulièrement avantageuse, l'extrait ou la composition nutraceutique ou cosmétique selon l'invention est utilisé dans des applications cosmétiques, avantageusement par voie topique, notamment pour le soin ou l'hygiène de la peau et/ou des muqueuses et/ou des phanères tels que les cheveux, ou encore pour la prévention et/ou le traitement des troubles de la peau et/ou des muqueuses et/ou des phanères tels que les cheveux.

### EXEMPLES

### Exemple 1. Procédé de préparation d'un extrait de tissus racinaires de C. sativa selon l'invention

Des graines de chanvre ont été stérilisées et mise en culture dans des boites de pétri composé d'un milieu de culture de germination (base MS). Après une semaine, les cultures ont été placés à la lumière (photo périodicité 8h - 16h).

Les plantules sont ensuite infectées par une bactérie du type *Agrobacterium rhizogenes.* Pour cela les plantules sont mises au contact de la bactérie pendant une semaine. Puis les plantules sont placées sur un milieu de sélection enrichi en source carboné. Les tissus racinaires commencent à apparaitre au bout de quelques jours, et sont repiqués sur un milieu de même composition jusqu'à l'obtention de tapis racinaires.

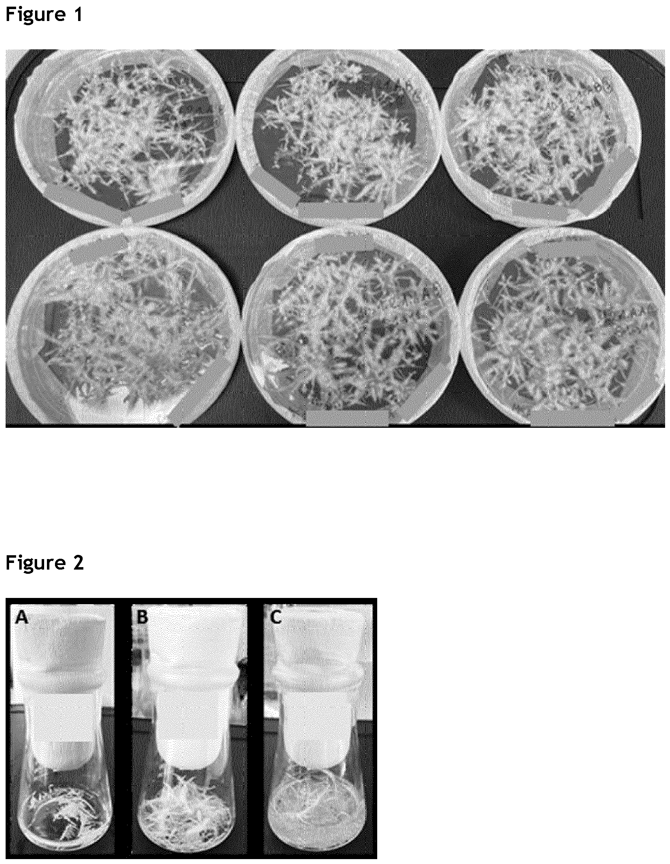

La figure 1 présente de tels tapis racinaires, visibles au bout de 3 semaines.

Afin d'amplifier les cultures, une partie du tapis racinaire est prélevé et mis en suspension dans un milieu de même composition sous agitation orbitale.

La figure 2 montre les récipients contenant cette biomasse en culture, après différents temps de culture. Au bout de 8 jours, on distingue nettement la structure de « chevelu racinaire ».

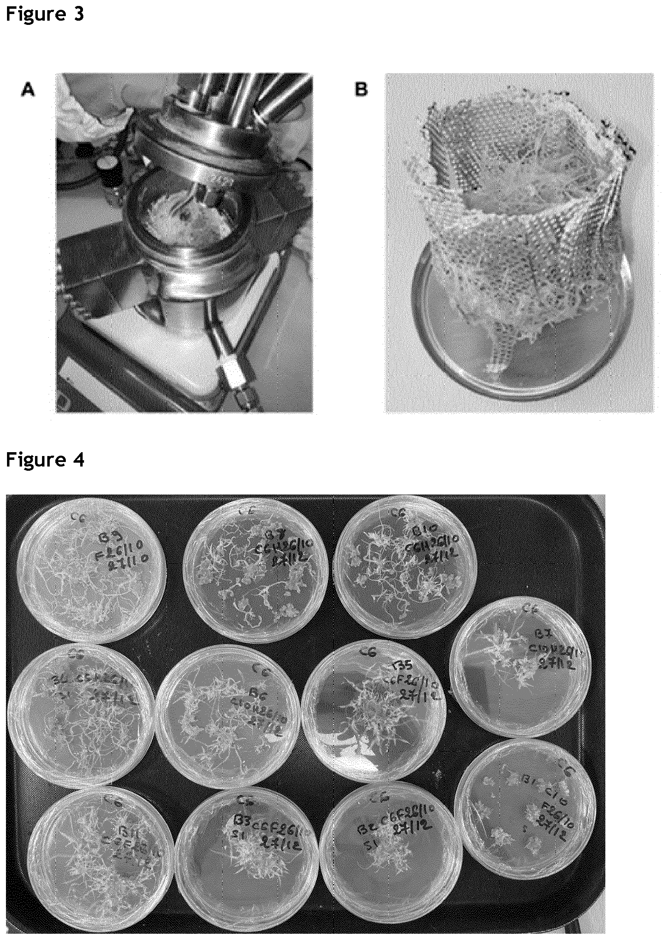

La figure 3 représente le bioréacteur utilisé (3A) ainsi que la biomasse constituée de chevelu racinaire récoltée à l'issue de la culture (3B).

Selon un autre procédé de l'invention, des explants de plantules sont prélevés, dédifférenciés et redifférenciés en tissus racinaires sur un milieu supplémenté en hormones végétales. Les tissus racinaires apparaissent au bout de de quelques jours de traitement, et sont repiqués sur un milieu de même composition jusqu'à l'obtention de tapis racinaires. Afin d'amplifier les cultures, une partie du tapis racinaire est prélevé et mis en suspension dans un milieu de même composition sous agitation orbitale.

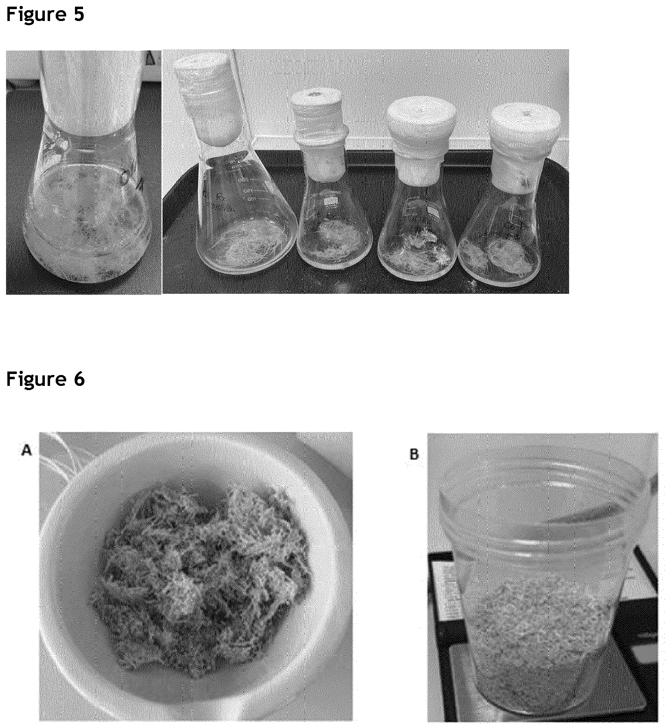

La figure 4 présente la culture de ces tissus racinaires en milieu solide, et la figure 5 montre ces cultures en milieu liquide.

A la fin de la culture, la biomasse est prélevée, séchée (figure 6A) et broyée au mortier (figure 6B).

### Exemple 2. Analyse de la composition biochimique de l'extrait de tissus racinaires de C. sativa cultivé in vitro

### A- Préparation de l'échantillon

L'échantillon est un extrait sec de tissus racinaires de *Cannabis sativa* cultivés *in vitro,* obtenu selon le procédé comprenant une transformation des tissus racinaires par *Agrobacterium rhizogenes.*

A partir de cet échantillon, deux extraits ont été préparé à l'aide d'un système de solvants biphasiques composé d'acétate d'éthyle, d'acétonitrile et d'eau en proportion 3 / 3/ 4 (v/v). Deux phases non miscibles ont ainsi été séparées : une phase apolaire contenant des molécules apolaires et une phase polaire contenant des molécules polaires. Pour obtenir un extrait sec permettant de procéder à la caractérisation biochimique, les deux phases ont ensuite été évaporées à l'évaporateur, séparément.

### B - Analyses RMN et identification des principaux métabolites

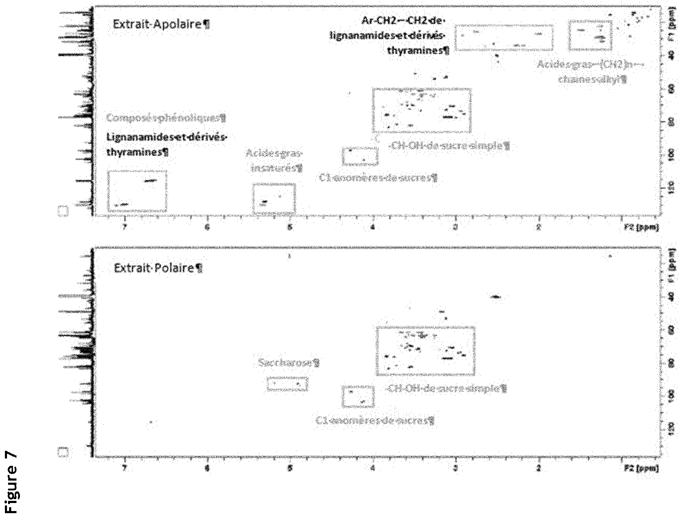

Chaque extrait a été analysé par RMN HSQC et les profils de RMN sont présentés dans la figure 7.

### C - Analyses par chromatographie liquide haute performance / spectrométrie de masse (HPLC/MS)

Les 2 extraits apolaire et polaire ont également été analysés par LC/MS en mode ion positif (ESI+) et négatif (ESI-).

Pour l'HPLC, deux phases mobiles différentes ont été utilisés selon l'extrait : une phase polaire a été utilisée pour l'extrait apolaire (par exemple H20 + 0.1% acide formique) et une non polaire pour l'extrait polaire (par exemple CH3CN + 0.1% acide formique).

Pour la spectrométrie de masse, les rapports masse/charges allant de 50 à 2000 m/z ont été observés.

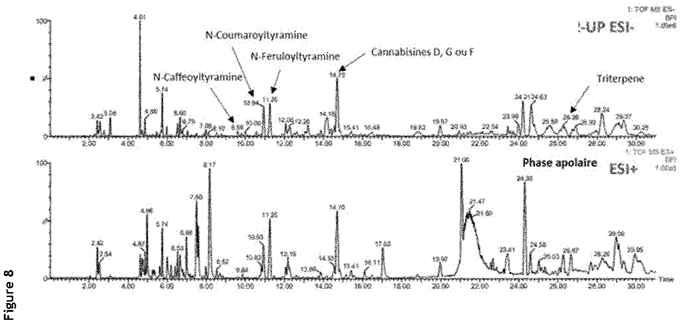

Les résultats de l'analyse biochimique par LC/MS des phases apolaire et polaire sont présentés dans le tableau 1 ci-dessous, ainsi que dans la figure 8.

**Tableau 1. Analyse biochimique par LC/MS**

| **LC temps de rétention (min)** | ***m*/*z* observé** | **Composition élémentaire** | **Δppm** | **Annotation** |
|---|---|---|---|---|
| **Extrait apolaire** | | | | |
| 2.42 | 311.1455 [M+H]⁺ | C11H23N2O8 | 0.3 | Glucopine, Mannopine ou isomère |
| | 309.1294 [M-H]⁻ | C11H21N2O8 | -1.3 | |
| 2.54 | 312.1295 [M+H]⁺ | C11H22NO9 | 0.0 | Acide Mannopinique ou isomère |
| 2.55 | 195.0500 [M-H]⁻ | C6H11O7 | -2.6 | Acide Gluconique ou isomère |
| 3.09 | 341.1085 [M-H]⁻ | C12H21O11 | 0.3 | Saccharose |
| 4.61 | 294.1194 [M+H]⁺ | C11H20NO8 | 1.0 | *Non assigné* |
| | 292.1029 [M-H]⁻ | C11H18NO8 | -1.0 | |
| 4.73 | 243.0618 [M-H]⁻ | C9H11N2O6 | 0.4 | Uridine |
| 4.74 | 268.1045 [M+H]⁺ | C10H14N5O4 | -0.1 | Adenosine |
| 4.82 | 282.0836 [M-H]⁻ | C10H12N5O5 | -0.7 | Guanosine |
| 4.96 | 180.1389 [M+H]⁺ | C11 H18NO | 0.6 | *Non assigné* |
| 5.25 | 253.1188 [M+H]⁺ | C12H17N2O4 | 0.0 | Dipeptide Phe-Ser ou isomère |
| 5.28 | 144.0485 [M+H]⁺ | ? | - | *Non assigné* |
| 5.36 | 270.2542 [M+H]⁺ | ? | - | *Non assigné* |
| 5.61 | 380.2914 [M+H]⁺ | C21H38N3O3 | 0.3 | *Non assigné* |
| 5.74 | 267.1345 [M+H]⁺ | C13H19N2O4 | 0.0 | |
| | 265.1189 [M-H]⁻ | C13H17N2O4 | 0.4 | Dipeptide Thr-Phe ou isomère |
| 5.98 | 299.0770 [M-H]⁻ | C13H15O8 | 1.0 | Hydroxybenzoyl glucose |
| 5.98 | 382.3071 [M+H]⁺ | C21H40N3O3 | 0.3 | Cannabisativine |
| 6.20 | 188.0713 [M+H]⁺ | C11H10NO2 | 0.5 | *Non assigné* |
| 6.41 | 284.2338 [M+H]⁺ | C15H30N3O2 | 0.0 | *Non assigné* |
| 6.54 | 233.1504 [M+H]⁺ | C10H21N2O4 | 1.3 | Dipeptide Thr-Leu ou isomère |
| | 231.1345 [M-H]⁻ | C10H19N2O4 | 0.0 | |
| 6.66 | 217.1554 [M+H]⁺ | C10H21N2O3 | 0.3 | |
| | 215.1400 [M-H]⁻ | C10H19N2O3 | 1.9 | Dipeptide Val-Val ou isomère |
| 6.79 | 215.1397 [M-H]⁻ | C10H19N2O3 | 0.5 | Dipeptide Val-Val ou isomère |
| 6.98 | 266.2232 [M+H]⁺ | ? | - | *Non assigné* |
| 7.05 | 249.1244 [M-H]⁻ | C13H17N2O3 | 2.0 | *Non assigné* |
| 7.22 | 467.1552 [M-H]⁻ | C22H27O11 | -0.2 | *Non assigné* |
| 7.50 | 236.0556 [M-H]⁻ | C11 H10NO5 | -1.3 | *Non assigné* |
| 7.50 | 268.2386 [M+H]⁺ | C15H30N3O | -1.1 | *Non assigné* |
| 7.59 | 326.2445 [M+H]⁺ | C17H32N303 | 0.3 | *Non assigné* |
| 7.87 | 444.1658 [M-H]⁻ | C23H26NO8 | 0.0 | N-Coumaroyltyramine glucoside |
| | 282.1129 [M-H-Glu]⁻ | C17H16NO3 | -0.4 | N-Coumaroyltyramine fragment |
| 7.98 | 476.1922 [M+H]⁺ | C24H30NO9 | 0.2 | N-Feruloyltyramine glucoside |
| | 474.1771 [M-H]⁻ | C24H28NO9 | 1.5 | N-Feruloyltyramine glucoside |
| | 312.1239 [M-H-Glu]⁻ | C18H18NO4 | 1.0 | N-Feruloyltyramine fragment |
| 8.10 | 474.1762 [M-H]⁻ | C24H28NO9 | -0.4 | N-Feruloyltyramine glucoside isomer2 |
| 8.20 | 328.2602 [M+H]⁺ | C17H34N3O3 | 0.6 | *Non assigné* |
| 8.53 | 488.1920 [M-H]⁻ | C25H30NO9 | -0.2 | *Non assigné* |
| 8.78 | 121.0290 [M-H]⁻ | C7H5O2 | 0.0 | Acide benzoique |
| 9.59 | 298.1076 [M-H]⁻ | C17H16NO4 | -1.0 | N-Caffeoyltyramine |
| 9.80 | 453.2337 [M-H]⁻ | C20H37O11 | 0.2 | *Non assigné* |
| 10.00 | 187.0966 [M-H]⁻ | C9H15O4 | -2.1 | Acide azelaique |
| 10.57 | 423.2235 [M-H]⁻ | C19H35O10 | 1.2 | *Non assigné* |
| 10.82 | 325.2277 [M+H]⁺ | C21H29N2O | -0.9 | *Non assigné* |
| 10.94 | 284.1288 [M+H]⁺ | C17H18NO3 | 0.4 | N-Coumaroyltyramine |
| | 282.1132 [M-H]⁻ | C17H16NO3 | 0.7 | |
| 11.26 | 314.1393 [M+H]⁺ | C18H20NO4 | 0.3 | N-Feruloyltyramine |
| | 312.1241 [M-H]⁻ | C18H18NO4 | 1.0 | |
| 12.06 | 625.2550 [M+H]⁺ | C36H37N2O8 | 0.0 | **Cannabisine D ou G ou F ou isomère** |
| | 623.2396 [M-H]⁻ | C36H35N2O8 | 0.5 | |
| 12.19 | 328.1548 [M+H]⁺ | C19H22NO4 | -0.3 | *Non assigné* |
| 12.27 | 625.2550 [M+H]⁺ | C36H37N2O8 | 0.0 | **Cannabisine D ou G ou F ou isomère** |
| | 623.2401 [M-H]⁻ | C36H35N2O8 | 1.3 | |
| 13.21 | 327.2178 [M-H]⁻ | C18H31O5 | 2.1 | Dérivé d'acide gras C18 |
| 14.18 | 329.2334 [M-H]⁻ | C18H33O5 | 1.8 | Dérivé d'acide gras C18 |
| 14.55 | 565.2342 [M+H]⁺ | C34H33N2O6 | 0.5 | *Non assigné* |
| 14.70 | 625.2556 [M+H]⁺ | C36H37N2O8 | 1.0 | **Cannabisine D ou G ou F ou isomère** |
| | 623.2394 [M-H]⁻ | C36H35N2O8 | 0.3 | |
| 15.41 | 639.2704 [M+H]⁺ | C37H39N2O8 | -0.3 | *Non assigné* |
| | 637.2552 [M-H]⁻ | C37H37N2O8 | 0.3 | |
| 16.11 | 316.2852 [M+H]⁺ | C18H38NO3 | 0.0 | Dérivé Sphingosine |
| 16.49 | 934.3574 [M-H]⁻ | ? | - | *Non assigné* |
| 17.03 | 318.3006 [M+H]⁺ | C18H40NO3 | -0.6 | Dérivé Sphingosine |
| 18.82 | 476.2780 [M-H]⁻ | ? | - | *Non assigné* |
| 19.99 | 603.3898 [M+H]⁺ | C35H55O8 | 0.2 | *Non assigné* |
| | 601.3741 [M-H]⁻ | C35H53O8 | 0.2 | |
| 24.21 | 471.3109 [M-H]⁻ | C29H43O5 | -0.2 | *Non assigné* |
| 24.34 | 481.2936 [M+H]⁺ | C30H41O5 | -3.7 | *Non assigné* |
| 24.63 | 271.2271 [M-H]⁻ | C16H31O3 | -0.7 | Acide Palmitique |
| 25.59 | 355.3218 [M-H]⁻ | C22H43O3 | 1.7 | Acide Hydroxydocosanoique |
| 26.26 | 455.3532 [M-H]⁻ | C30H47O3 | 1.5 | Triterpène (oleanolique ou ursolique ou isomère) |
| 26.90 | 299.2593 [M-H]⁻ | C18H35O3 | 2.3 | Dérivé d'acide gras C18 |
| 28.22 | 605.4058 [M-H]⁻ | C36H53N4O4 | -1.5 | *Non assigné* |

| **Extrait polaire** | | | | |
|---|---|---|---|---|
| 2.42 | 311.1455 [M+H]⁺ | C11H23N2O8 | 0.3 | Glucopine or Mannopine ou isomère |
| | 309.1294 [M-H]⁻ | C11H21N2O8 | -1.3 | |
| 2.54 | 312.1295 [M+H]⁺ | C11H22NO9 | 0.0 | Acide Mannopinique ou isomère |
| 2.55 | 195.0500 [M-H]⁻ | C6H11O7 | -2.6 | Acide Gluconique ou isomère |
| 2.77 | 404.1041 [M-H]⁻ | C12H22NO14 | 0.2 | *Non assigné* |
| 3.09 | 341.1085 [M-H]⁻ | C12H21O11 | 0.3 | Saccharose |
| 3.18 | 253.0926 [M-H]⁻ | C9H17O8 | 1.2 | Hexoside de Glycerol |
| 3.91 | 606.0735 [M-H]⁻ | C25H20NO17 | 0.7 | *Non assigné* |
| 4.61 | 294.1194 [M+H]⁺ | C11H20NO8 | 1.0 | *Non assigné* |
| | 292.1029 [M-H]⁻ | C11H18NO8 | -1.0 | |
| 4.82 | 282.0836 [M-H]⁻ | C10H12N5O5 | -0.7 | Guanosine |
| 5.74 | 267.1345 [M+H]+ | C13H19N2O4 | 0.0 | Dipeptide Thr-Phe ou isomère |
| | 265.1189 [M-H]- | C13H17N2O4 | 0.4 | |
| 6.54 | 233.1504 [M+H]+ | C10H21N2O4 | 1.3 | Dipeptide Thr-Leu ou isomère |
| | 231.1345 [M-H]- | C10H19N2O4 | 0.0 | |
| 6.66 | 217.1554 [M+H]+ | C10H21N2O3 | 0.3 | Dipeptide Val-Val ou isomère |
| | 215.1400 [M-H]- | C10H19N2O3 | 1.9 | |
| 6.98 | 266.2232 [M+H]+ | ? | - | *Non assigné* |
| 7.50 | 236.0556 [M- | C11H10NO5 | -1.3 | *Non assigné* |
| 7.50 | 268.2386 [M+H]+ | C15H30N3O | -1.1 | *Non assigné* |
| 8.20 | 328.2602 [M+H]+ | C17H34N3O3 | 0.6 | *Non assigné* |

Les résultats présentés dans le tableau 1 mettent en évidence la présence des composés d'intérêt suivants dans l'extrait selon l'invention :
- Des acides phénoliques dont des triterpènes, notamment de l'acide oléanolique et de l'acide ursolique ;
- Stérols, notamment beta-sitostérol,
- Acides gras, saturés et non saturés,
- Nucléosides, di-peptides et
- Un grand nombre d'alcaloïdes dont la cannabisativine et des dérivés de tyramine.

Parmi des dérivés de tyramine, on note en particulier la présence de cannabisines, probablement de cannabisine D, F ou G. Sont également présents dans l'extrait selon l'invention les composés suivants : N-coumaroyltyramine, N-feruloyltyramine et N-caffeoyltyramine.

### D - Dosage des triterpènes et des cannabisines de l'extrait de l'invention

Le taux de triterpènes présent dans l'extrait est mesuré par Chromatographie en Phase Gazeuse couplée à un détecteur à Ionisation de Flamme (GC-FID).

Les résultats concernant le dosage des triterpènes suivants : beta amyrine, épifriedelanol et friedéline, sont présentés dans le tableau 2 ci-dessous.

Deux extraits ont été analysés : (i) l'extrait de tissus racinaires selon l'invention, et (ii) un extrait de racines naturelles, obtenu par un procédé comprenant le prélèvement de plantes cultivées en champ, puis le lavage, le broyage et le séchage des racines.

**Tableau 2 : Dosage des triterpènes par GC-FID**

| | βamyrine | Epifriedelanol | Friedeline |
|---|---|---|---|
| Extrait de tissus racinaires selon l'invention | 110 µg/g | 670 µg/g | 1,63mg/g |
| Extrait de racines naturelles | ≤15 µg/g | ≤15 µg/g | ≤50 µg/g |

Le taux des cannabisines D et F et de la grossamide est mesuré par chromatographie liquide couplée à un détecteur à temps de vol haute résolution (LC QToF).

Les résultats de dosage de ces trois cannabisines particulières, en comparaison avec le taux observé dans un extrait de racines naturelles, sont présentés dans le tableau 3 ci-dessous.

**Tableau 3 : Dosage des cannabisines par LC QToF**

| | Canabisine D / 1 g extrait sec | Cannabisine F + Grossamide / 1 g extrait sec |
|---|---|---|
| Extrait de tissus racinaires selon l'invention | 72,3 µg/g | 311,1 µg/g |
| Extrait de racines naturelles | ≤5 µg/g | ≤10 µg/g |

La cannabisine F est mesurée conjointement avec la grossamide car lors de l'analyse, leurs pics étaient superposés.

L'extrait selon l'exemple comprend 383,4 µg de cannabisines pour 1 gramme d'extrait sec, donc représente 0,038 % en poids de l'extrait total.

Ces résultats démontrent que l'extrait de tissus racinaires selon l'invention est riche en lignanamides et notamment en cannabisines. De plus, le taux relatif de cannabisines est nettement supérieur à celui observé dans des racines naturelles de *Cannabis sativa.*

### Exemple 3. Applications de l'extrait selon l'invention

Pour cette étude, le potentiel antimicrobien/antioxydant a été évalué pour les échantillons STH1 (extrait de racine selon l'invention) et STH2 (extrait de racine naturelle) ainsi que 14 autres échantillons standards issue d'une extractothéque utilisé comme témoin et pour les analyses statistiques. Les échantillons repris à une concentration de 5mg/mL dans du DMSO, ont été confrontés à un set de protéines permettant d'évaluer ces activités, à raison de 400µg en milieu réactionnel aqueux.

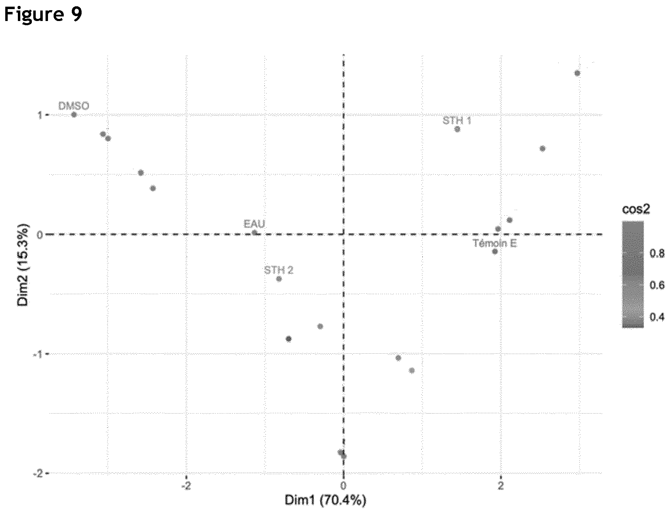

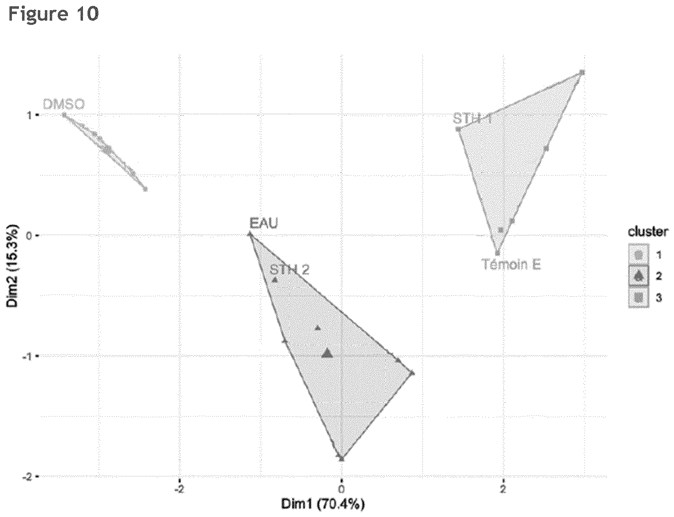

Les résultats obtenus ont ensuite été traités statistiquement par une analyse en composante principale (ACP) puis une clusterisation hiérarchique ascendante effectuée avec le logiciel R. Les résultats obtenus sont présentés respectivement en figures 9 et 10.

La figure 9 matérialise un gradient de réactivité, les échantillons les moins réactifs étant situés dans la partie gauche du graphique, proche du témoin (solvant DMSO). Plus les échantillons sont dispersés sur la partie droite et plus ces derniers sont réactifs et présentent donc un potentiel antimicrobien/antioxydant. Le témoin E (témoin positif) est un extrait connu pour ses propriété antimicrobienne et antioxydante et sa forte réactivité avec ce test *in vitro.*

La seconde analyse présentée en figure 10 permet de former, d'un point de vue statistique, 3 grands groupes au sein de l'ACP. Le premier groupe présent à gauche, comporte le témoin solvant DMSO et les échantillons n'ayant aucune réactivité avec le système. Le groupe 3 en haut à droite, est composé des échantillons ayant une très forte réactivité avec le système. Enfin, le groupe 2 au centre, comprend une réactivité intermédiaire avec les protéines. De façon intéressante, nous pouvons observer que les échantillons STH1 et STH2 se trouvent dans le groupe 3 et le groupe 2 respectivement, indiquant que les échantillons analysés présentent un fort potentiel antimicrobien/antioxydant pour l'échantillon STH1 et un potentiel modéré pour l'échantillon STH2.

### A - Mise en évidence de l'activité antimicrobienne

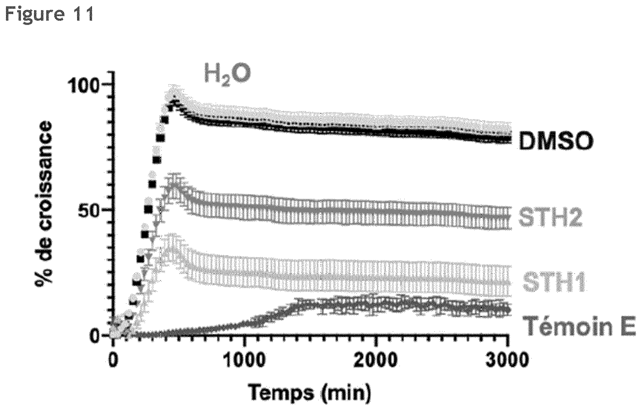

Dans le but d'illustrer le potentiel antimicrobien des deux extraits STH1 et STH2, il a été mené une évaluation de la capacité de ces derniers à inhiber la croissance d'un champignon unicellaire *Saccharomyces cereviseae* dans des conditions optimales de croissance durant 48h à une concentration de 1mg/mL. Les courbes de croissance ainsi que le tableau indiquant les pourcentages d'inhibition sont présentés en figure 11.

Les capacités d'inhibition des extraits sont résumées dans le tableau 4 ci-dessous.

**Tableau 4 : Pourcentage d'inhibition des témoins négatif (H20 et DMSO) et positif (Témoin E) et des échantillons STH1 et STH2**

| Echantillons | Pourcentage d'inhibition |
|---|---|
| H20 | 0% |
| DMSO | 2% |
| STH1 (extrait selon l'invention) | 61% |
| STH2 (extrait de racine naturelle) | 32% |
| Témoin E | 79% |

En ce qui concerne l'échantillon STH1 on note une forte inhibition de croissance de 61% et pour l'échantillon STH2, une inhibition modérée de 32%.

### B - Mise en évidence de l'activité antioxydante

Par ailleurs, pour illustrer le potentiel antioxydant des deux extraits STH1 et STH2, il a été mené un test anti-radicalaire. Le protocole consiste à réaliser une gamme étalon d'acide gallique, utilisé en tant qu'agent oxydant, en réaction à une solution d'ABTS (2,2'-azinobis[3-éthylbenzothiazoline-6-sulfonique]) dont l'intensité de couleur varie en fonction de l'activité. Un suivi d'absorbance à 734 nm a été réalisé, à température ambiante, en microplaque 96 puits.

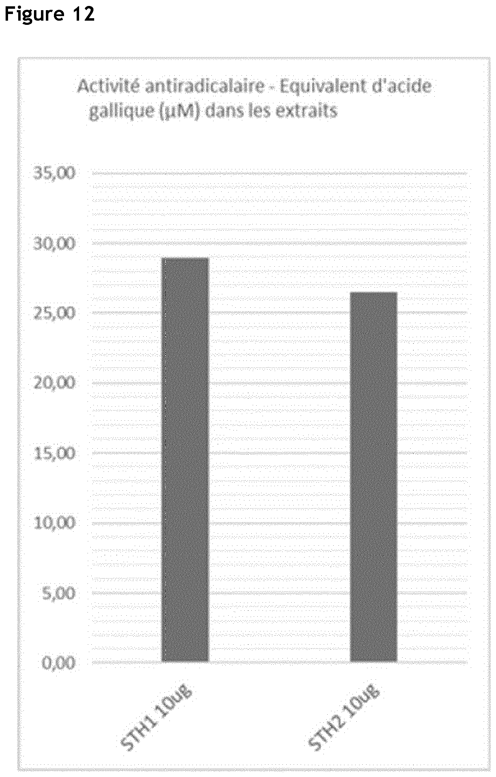

Les résultats sont présentés dans la figure 12, en unité d'équivalent d'acide gallique (µM).

Le dosage d'activité antiradicalaire indique une réactivité équivalente à 30µM d'acide gallique pour 10µg d'extrait STH1. On observe une meilleure activité de STH1 (extrait selon l'invention) comparé à STH2 (extrait de racine naturelle).

### REFERENCES BIBLIOGRAPHIQUES

Brenneisen, Chemistry and Analysis of Phytocannabinoids and Other Cannabis Constituents Rudolf Brenneisen, 2007) (livre)
Sakakibara I, Ikeya Y, Hayashi K, Okada M, Maruno M. Three acyclic bis-phenylpropane lignanamides from fruits of Cannabis sativa. Phytochemistry. 1995 Mar;38(4):1003-7. doi: 10.1016/0031-9422(94)00773-m. PMID: 7766383.
Flores-Sanchez, I.J., Verpoorte, R. Secondary metabolism in cannabis. Phytochem Rev 7, 615-639 (2008).
William Leonard, Pangzhen Zhang, Danyang Ying Σε Zhongxiang Fang (2021) Lignanamides: sources, biosynthesis and potential health benefits - a minireview, Critical Reviews in Food Science and Nutrition, 61:8, 1404-1414.
Farag S. Σε Kayser O. "Cannabinoids production by hairy root cultures of Cannabis sativa L." American Journal of Plant Sciences 6.11 (2015): 1874.
Yan X, Tang J, dos Santos Passos C, Nurisso A, Simôes-Pires CA, Ji M, Lou H, Fan P. Characterization of Lignanamides from Hemp (Cannabis sativa L.) Seed and Their Antioxidant and Acetylcholinesterase Inhibitory Activities. J Agric Food Chem. 2015 Dec 16;63(49):10611-9. doi: 10.1021/acs.jafc.5b05282. Epub 2015 Dec 2. PMID: 26585089.
Ryz NR, Remillard DJ, Russo EB. Cannabis Roots: A Traditional Therapy with Future Potential for Treating Inflammation and Pain. Cannabis Cannabinoid Res. 2017 Aug 1;2(1):210-216. doi: 10.1089/can.2017.0028. PMID: 29082318; PMCID: PMC5628559.
Kornpointner, C., Martinez, A. S., Marinovic, S., Haselmair-Gosch, C., Jamnik, P., Schröder, K., ... & Halbwirth, H. (2021). Chemical composition and antioxidant potential of Cannabis sativa L. roots. Industrial Crops and Products, 165, 113422.
Wang, S.; Luo, Q.; Fan, P. Cannabisin F from Hemp (Cannabis sativa) Seed Suppresses Lipopolysaccharide-Induced Inflammatory Responses in BV2 Microglia as SIRT1 Modulator. Int. J. Mol. Sci. 2019, 20, 507. https://doi.org/10.3390/ijms20030507
Wahby, I., Caba, J. M., & Ligero, F. (2013). Agrobacterium infection of hemp (Cannabis sativa L.): establishment of hairy root cultures. Journal of Plant Interactions, 8(4), 312-320.
Berahmand F, Beizaee N, Dehghan Nayyeri M, Sharafi A, Kheiri Manjili H, Danafar H et al . Cannabis sativa L. genetically transformed root based culture via Agrobacterium rhizogenes. Pharm Biomed Res 2016; 2 (3) :13-1.

## Revendications

1. Extrait de tissus racinaires de C. *sativa* cultivés *in vitro,* **caractérisé en ce que** ledit extrait est constitué de la biomasse desdits tissus racinaires, et comprend au moins 0,01% en poids de lignanamide(s) par rapport au poids de l'extrait sec.

2. Extrait de tissus racinaires de C. *sativa* selon la revendication 1, **caractérisé en ce que** au moins une lignanamide est une cannabisine, en particulier choisie parmi les cannabisines A, B, C, D, E, F et G.

3. Extrait de tissus racinaires de C. *sativa* selon l'une des revendications 1 ou 2, **caractérisé en ce que** ladite biomasse est séchée et broyée.

4. Extrait de tissus racinaires de C. *sativa* selon l'une des revendication 1 à 3, **caractérisé en ce que** les tissus racinaires sont du chevelu racinaire de C. *sativa,* séché et broyé.

5. Procédé de préparation d'un extrait de tissus racinaires de C. *sativa* selon la revendication 4, comprenant la transformation de plantules avec une bactérie *Agrobacterium rhizogenes* pour l'obtention d'un chevelu racinaire, cultivé dans un milieu nutritif, puis séché et broyé.

6. Procédé de préparation d'un extrait de tissus racinaires de C. *sativa* selon l'une des revendications 1 à 3, comprenant la dédifférentiation d'un explant de C. sativa en cal, puis la différentiation de ce cal en tissus racinaires adventives, cultivés dans un milieu nutritif, puis séchés et broyés.

7. Composition pharmaceutique comprenant en tant qu'actif un extrait de tissus racinaires de C. *sativa* selon l'une revendications 1 à 4 ou tel qu'obtenu par le procédé de l'une des revendications 5 ou 6.

8. Composition dermatologique, comprenant en tant qu'actif un extrait de tissus racinaires de C. *sativa* selon l'une revendications 1 à 4 ou tel qu'obtenu par le procédé de l'une des revendications 5 ou 6.

9. Composition nutraceutique comprenant en tant qu'actif un extrait de tissus racinaires de C. *sativa* selon l'une revendications 1 à 4 ou tel qu'obtenu par le procédé de l'une des revendications 5 ou 6.

10. Composition cosmétique comprenant en tant qu'actif un extrait de tissus racinaires de C. *sativa* selon l'une revendications 1 à 4 ou tel qu'obtenu par le procédé de l'une des revendications 5 ou 6.

11. Composition vétérinaire comprenant en tant qu'actif un extrait de tissus racinaires de C. *sativa* selon l'une revendications 1 à 4 ou tel qu'obtenu par le procédé de l'une des revendications 5 ou 6.

12. Extrait de tissus racinaires de C. *sativa* selon l'une des revendications 1 à 4, ou composition pharmaceutique selon la revendication 7, ou composition dermatologique selon la revendication 8, pour son utilisation dans la prévention et/ou le traitement des réactions, troubles ou pathologies de la peau et/ou des muqueuses et/ou des phanères.

13. Extrait selon l'une des revendications 1 à 4, ou composition pharmaceutique selon la revendication 7, ou composition dermatologique selon la revendication 8, pour son utilisation selon la revendication 12, **caractérisée en ce que** les réactions ou pathologies sont de type allergique, inflammatoire ou irritative.

14. Utilisation nutraceutique ou cosmétique d'un extrait de tissus racinaires de C. *sativa* selon l'une des revendications 1 à 4, ou d'une composition nutraceutique selon la revendication 9, ou d'une composition cosmétique selon la revendication 10 ou d'une composition vétérinaire selon la revendication 11.

15. Procédé de soin cosmétique de la peau et/ou des phanères et/ou des muqueuses d'un Homme ou d'un animal, en vue d'améliorer leur état et/ou leur aspect, comprenant l'administration ou l'application d'une composition nutraceutique selon la revendication 9 ou d'une composition cosmétique selon la revendication 10 ou d'une composition vétérinaire selon la revendication 11.
